# EUROPÄISCHE PATENTSCHRIFT

(11) **EP 0 538 183 B1**
(45) Veröffentlichungstag und Bekanntmachung des Hinweises auf die Patenterteilung: **31.01.1996**
(21) Anmeldenummer: 92810584.0
(22) Anmeldetag: 31.07.1992
(51) Int. Cl.: A61F 2/44

(54) **Bandscheibenprothese**
Prosthetic intervertebral disc
Prothèse de disque intervertébral

(30) Priorität: 30.08.1991 CH 2552/91
(43) Veröffentlichungstag der Anmeldung: 21.04.1993
(73) Patentinhaber: SULZER Medizinaltechnik AG, CH-8404 Winterthur (CH)
(72) Erfinder: Baumgartner, Walter, CH-9500 Wil (CH)
(74) Vertreter: Hammer, Bruno, Dr.

(56) Entgegenhaltungen:
- FR-A- 2 124 815
- US-A- 4 714 469

## Beschreibung

Die Erfindung handelt von einer Bandscheibenprothese, die in ihren äusseren Abmessungen einer natürlichen Bandscheibe nachgeformt ist, die im implantierten Zustand in ihrer Achse zwei benachbarte Wirbel verbindet und die eine obere und eine untere Aufnahmefläche aufweist, wobei der Bandscheiben Körper einteilig ist und aus einem festen, elastisch deformierbaren Werkstoff besteht. Eine solche Bandscheibenprothese ist aus der FR-A-2 124 815 bekannt.

Die Patentschrift US 4,309,777 zeigt eine Bandscheibenprothese, die aus einer Dose mit einer unteren und einer oberen Hälfte, die unter Kompression von eingelegten Schraubenfedern gegeneinander beweglich sind. Diese Lösung hat den Nachteil, dass die aneinander gleitenden Dosenränder bei Biegebelastungen verklemmen können, dass Abrieb erzeugt wird und dass in der Dose ein eingeschlossenes relativ grosses Leervolumen besteht, welches sich mit Körperflüssigkeit füllen kann.

Hier schafft die Erfindung Abhilfe. Sie löst die Aufgabe eine einfache Bandscheibenprothese zu schaffen, die die vorher erwähnten Nachteile nicht aufweist. Gemäss der Erfindung wird die Aufgabe dadurch gelöst, dass der Bandscheibenkörper quer zur Achse parallele und sich teilweise überdeckende Schlitze aufweist, wobei sich benachbarte Schlitze in einem Ueberdeckungsbereich Teile von Blattfedern zur Uebertragung von Kräften von einer Aufnahmefläche zur anderen Aufnahmefläche bilden.

Die Vorteile der Erfindung sind darin zu sehen, dass die Prothese einfach in ihrer Handhabung ist und dass sie bei Biegebelastung und bei Druckbelastung eine nicht lineare Federcharakteristik besitzt, die in einer Wegbegrenzung endet. Die abhängigen Unteransprüche 2 bis 12 beziehen sich auf vorteilhafte Weiterbildungen der Erfindung.

Im folgenden wird die Erfindung anhand von Ausführungsbeispielen beschrieben. Es zeigen:
- Figur 1: die Draufsicht auf eine erfindungsgemässe Bandscheibenprothese, die Blattfedern mit drei Einspannzonen aufweist;
- Figur 2: die Seitenansicht eines Schnitts durch die Bandscheibenprothese von Fig. 1;
- Figur 3: die Frontalansicht eines Schnitts durch die Bandscheibenprothese von Fig. 1;
- Figur 4: die Draufsicht auf eine erfindungsgemässe Bandscheibenprothese, die Blattfedern mit zwei Einspannzonen aufweist;
- Figur 5: die Seitenansicht eines Schnitts durch die Bandscheibenprothese von Fig. 4;
- Figur 6: die Frontalansicht eines Schnitts durch die Bandscheibenprothese von Fig. 4;
- Figur 7: die Draufsicht auf eine erfindungsgemässe Bandscheibenprothese, die Blattfedern mit zwei Einspannzonen und Blattfedern mit einer Einspannzone aufweist;
- Figur 8: die Seitenansicht eines Schnitts durch die Bandscheibenprothese von Fig. 7;
- Figur 9: die Frontalansicht eines Schnitts durch die Bandscheibenprothese von Fig. 7;
- Figur 10: die nicht lineare Federcharakteristik bei vertikaler Kompression der Bandscheibenprothese (Druckkraft in Funktion von Auslenkung);
- Figur 11: die nicht lineare Federcharakteristik bei lateraler und bei frontaler Flexion der Bandscheibenprothese (Biegemoment in Funktion von Biegewinkel).

In den Figuren ist eine Bandscheibenprothese gezeigt, die in ihren äusseren Abmessungen einer natürlichen Bandscheibe nachgeformt ist und zwei benachbarte Wirbel mit ihrer oberen und unteren Aufnahmefläche verbindet. Der Bandscheibenkörper ist einteilig, besteht aus einem festen, elastisch deformierbaren Werkstoff und weist quer zur verbindenden Achse parallele und sich teilweise überdeckende Schlitze auf. Dabei bilden sich benachbarte Schlitze in einem Ueberdeckungsbereich Teile von Blattfedern zur Uebertragung von Kräften von einer Aufnahmefläche zur anderen Aufnahmefläche.

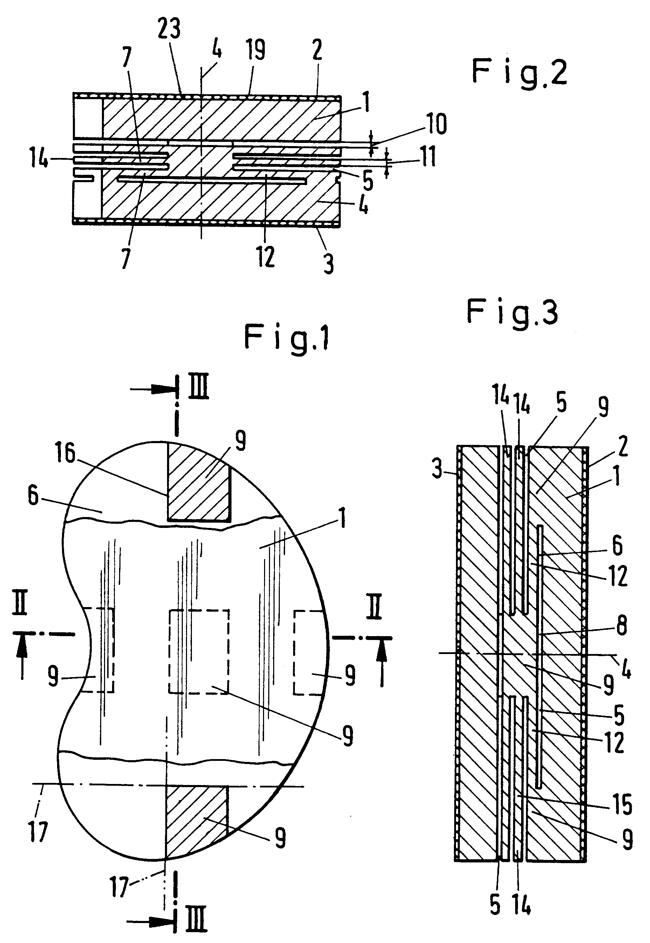

In einer Ausführung nach den Figuren 1, 2, 3 ist ein Bandscheibenkörper 1 auf seiner oberen Begrenzungsfläche 2 und auf seiner unteren Begrenzungsfläche 3 mit einer strukturierten Oberfläche 19 versehen, die beispielsweise aus einem Gitter besteht. Quer zur vertikalen Achse 4 sind im Bandscheibenkörper 1 Schlitze 5 angebracht, die den Bandscheibenkörper 1 aber nie ganz entzweischneiden. Diese Schlitze 5 sind parallel zueinander, überdecken sich teilweise und bilden mit ihren Begrenzungsflächen 8 - wenn es sich um benachbarte Schlitze 5 handelt - Teile von Blattfedern 7 im Ueberdeckungsbereich 6. Im sich nicht überdeckenden Bereich der benachbarten Schlitze 5 ist die Federwirkung der Blattfedern 7 durch Einspannzonen 9 unterbrochen. Die an der Bandscheibe angreifenden Kräfte werden von einer Blattfederebene zur nächsten Blattfederebene über die Einspannzonen 9 übertragen.

In Figur 3 sind in der obersten Blattfederebene drei Einspannzonen 9 an der Blattfeder 12, zwei äussere Einspannzonen 9 mit Uebergang zum Oberteil und eine mittlere Einspannzone 9 mit Uebergang zur nächsten tieferen Blattfederebene, in der nur eine mittlere Einspannzone 9 an Blattfeder 15 vorhanden ist. In Figur 2 sind in der untersten Blattfederebene drei Einspannzonen 9 zu sehen. Die Blattfedern mit drei Einspannzonen werden hier auf Biegung und Zug beansprucht, während die mittlere Blattfeder 15 bei Biegung eine Zusatzkraft erzeugt, wenn ihr freies Ende 14 einseitig anliegt und als Wegbegrenzung wirkt, wenn ihr freies Ende 14 beidseitig anliegt. Ebenso wirken die freien Enden 14 der anderen Blattfedern als Zusatzwiderstand und als Wegbegrenzung.

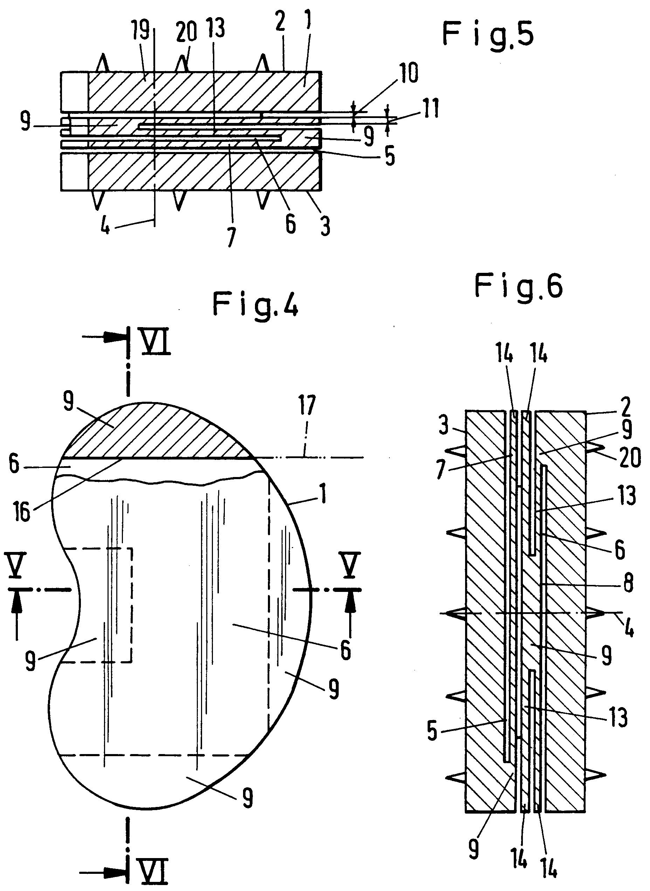

In einer Ausführung nach den Figuren 4, 5, 6 sind strukturierte Oberflächen 19 mit Spitzen 20 und Blattfedern 13 mit zwei Einspannzonen 9 gezeigt. Die Schlitze 5 sind jeweils von einer Seite her eingeschnitten, wobei die Erzeugende 17 eines Schlitzes 5 im rechten Winkel zu der Erzeugenden 17 des Nachbarschlitzes 5 steht. Die von medial entgegengesetzt eingeschnittenen Schlitze 5 sind abgesehen von den versetzten Ebenen symmetrisch zueinander, um bei zentrischer Druckbelastung keine seitliche Biegung zwischen der oberen Aufnahmefläche 2 und der unteren Aufnahmefläche 3 zu erzeugen. Schlitzbreite 10 und Blattfederdicke 11 sind so gewählt, dass beim Ueberschreiten bestimmter Druck- und/oder Biegebelastungen die Schlitzbreite 10 beidseitig der Blattfedern an bestimmten Orten auf Null reduziert wird und Wegbegrenzungen entstehen, bevor an den Blattfedern die Elastizitätsgrenze erreicht ist.

Die Herstellung solcher Bandscheibenkörper hängt vom gewählten Werkstoff ab, der genügend Festigkeit und ein elastisches Verhalten aufweisen muss. Bei Metallen wie z.B. Titanlegierungen können die Schlitze 5 durch Sägen oder funkenerosives Drahtschneiden erzeugt werden, wobei eine tangentiale Erzeugende 17 im Grund 16 des Schlitzes anliegt. Eine weitere Möglichkeit besteht im Schichten und Verbinden von Blattfedern, die in den Einspannzonen 9 der Schlitzbreite 10 entsprechende Distanzierstücke erhalten. Zum Fügen eines solchen Körpers kommen bei Metallen metallurgische Verbindungstechniken in Frage, während bei Kunststoffen und Faserverbundwerkstoffen Kleber und Binder in Frage kommen, die eine entsprechend hohe Affinität zum Grundmaterial aufweisen. Von den Einschränkungen bezüglich fügen ist die strukturierte Oberfläche 19 ausgenommen, die im Beispiel der Figuren 1, 2, 3 ein Metallgitternetz 23 ist, das sowohl mit einem metallischen Bandscheibenkörper 1 verschweisst sein kann, als auch von einem Bandscheibenkörper 1 aus Kunststoff teilweise umschlossen sein kann.

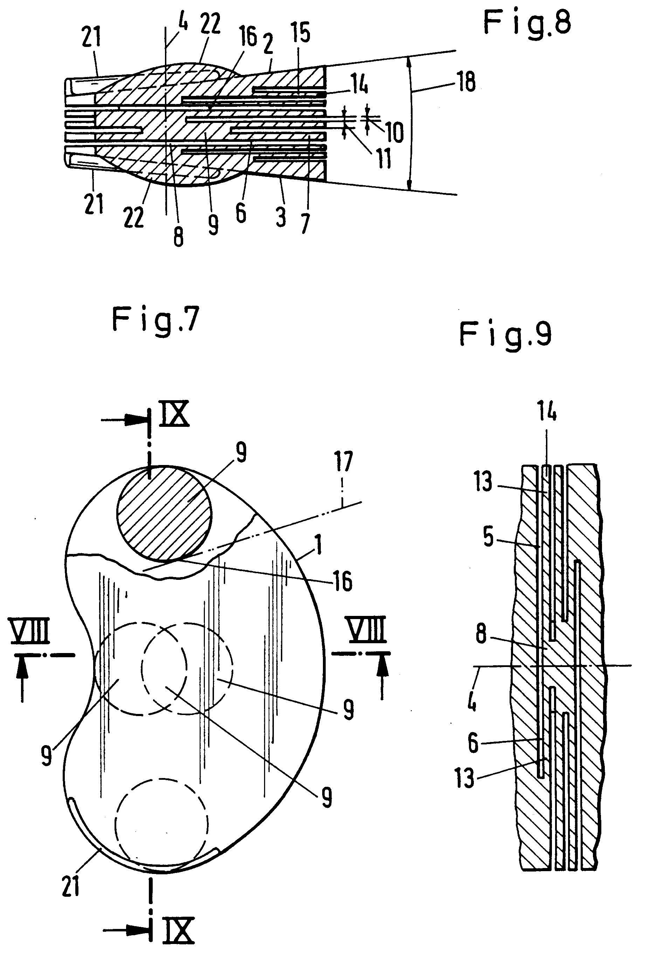

In einer Ausführung nach den Figuren 7, 8, 9 laufen die Aufnahmefläche von ventral nach dorsal keilförmig mit einem Keilwinkel 18 aufeinander zu. Ein Teil der Aufnahmeflächen 2, 3 ist konvex ausgeführt, damit er in intraoperativ erzeugten konkaven Gegenflächen, die nicht gezeigt sind, verankert werden kann. Als zusätzliche Verankerungshilfe sind in Figuren 7, 8 Teile eines zum Nachbarwirbel vorstehenden Kragens 21 gezeigt, die ein Verrutschen der Bandscheibenprothese verhindern sollen. Die Einspannzonen 9 sind kreisförmig ausgeführt, sodass eine tangentiale Erzeugende 17 nur noch punktförmig im Schlitzgrund 16 anliegt und um den Mittelpunkt der kreisförmigen Einspannzone 9 rotiert werden kann. Der Vorteil von runden Einspannzonen 9 besteht in einer richtungsunabhängigen Krafteinleitung in die Biegefedern 7. In Figur 8 sind zusätzlich Blattfedern 15 gezeigt, die als Einspannzone 9 nur einen ausgeprägten Ort besitzen, indem einer der begrenzenden Schlitze 5 nur von einer Seite eingeschnitten ist. Wird bei dieser Feder 15 eine Nachbarfeder gegen das freie Ende 14 gepresst, baut sich ein zusätzlicher Widerstand auf bis beim Anschlagen an der Begrenzungsfläche 8 im Schlitz auf der anderen Seite eine Wegbegrenzung entsteht.

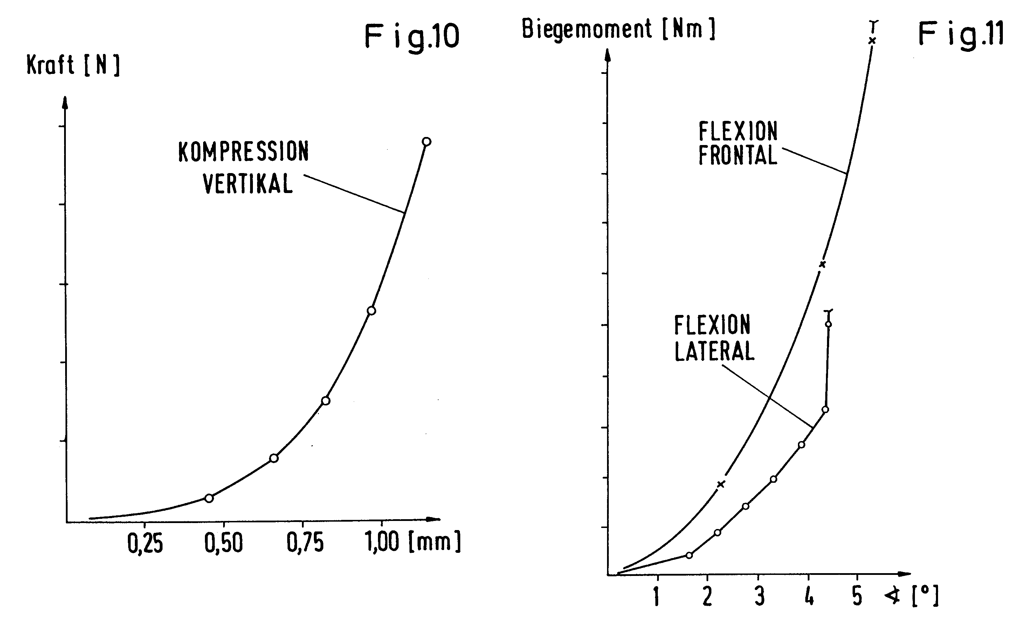

Bandscheibenkörper 1, können mit nicht linearen Charakteristiken für Kompression und Biegung ausgestattet werden, wenn im Verlauf der Deformation von Blattfedern zusätzliche Blattfedern in Eingriff kommen oder zusätzliche Abstützpunkte durch die Deformation entstehen. Typische Messresultate für Kompression und für Flexion einer Bandscheibenprothese sind in den Diagrammen der Figuren 10 und 11 gezeigt. Figur 10 zeigt die Abhängigkeit zwischen einer in Achse 4 angreifenden Druckkraft in Newton und der Kompression des Bandscheibenkörpers in Millimeter. Figur 11 zeigt das Biegemoment in Newton Meter für verschiedene Biegewinkel in ^{o} bei Biegung in frontaler Richtung und bei Biegung in seitlicher Richtung.

## Patentansprüche

1. Bandscheibenprothese, die in ihren äusseren Abmessungen einer natürlichen Bandscheibe nachgeformt ist, die im implantierten Zustand in ihrer Achse (4) zwei benachbarte Wirbel verbindet und die eine obere und eine untere Aufnahmefläche (2, 3) aufweist, wobei der Bandscheibenkörper (1) einteilig ist und aus einem festen, elastisch deformierbaren Werkstoff besteht, **dadurch gekennzeichnet,** dass der Bandscheibenkörper (1) quer zur Achse (4) parallele und sich teilweise überdeckende Schlitze (5) aufweist, wobei sich benachbarte Schlitze (5) in einem Ueberdeckungsbereich (6) Teile von Blattfedern (7, 12, 13, 15) zur Uebertragung von Kräften von der oberen Aufnahmefläche (2) zur unteren Aufnahmefläche (3) bilden.

2. Bandscheibenprothese entsprechend Anspruch 1, dadurch gekennzeichnet, dass Schlitzbreite (10) und Blattfederdicke (11) so gewählt sind, dass beim Ueberschreiten bestimmter Druck- und/oder Biegebelastungen die Schlitzbreite (10) an bestimmten Orten auf Null reduziert wird und Wegbegrenzungen entstehen bevor in den Blattfedern die Elastizitätsgrenze erreicht ist.

3. Bandscheibenprothese nach einem der Ansprüche 1 oder 2, dadurch gekennzeichnet, dass der feste, elastisch deformierbare Werkstoff Metall ist.

4. Bandscheibenprothese nach einem der Ansprüche 1 bis 3, dadurch gekennzeichnet, dass die Blattfeder (12) einer Ebene an drei ausgeprägten Orten eingespannt ist.

5. Bandscheibenprothese nach einem der Ansprüche 1 bis 3, dadurch gekennzeichnet, dass die Blattfeder (13) einer Ebene an zwei ausgeprägten Orten eingespannt ist.

6. Bandscheibenprothese nach einem der Ansprüche 1 bis 3, dadurch gekennzeichnet, dass die Blattfeder (15) einer Ebene an einem ausgeprägten Ort eingespannt ist.

7. Bandscheibenprothese nach einem der Ansprüche 1 bis 6, dadurch gekennzeichnet, dass die freien Enden (14) von Blattfedern (7) bei einseitiger Reduktion der Schlitzbreite (10) auf Null als Biegefedern wirken und bei beidseitiger Reduktion der Schlitzbreite (10) auf Null als Wegbegrenzung wirken.

8. Bandscheibenprothese nach einem der Ansprüche 1 bis 6, dadurch gekennzeichnet, dass Schlitze (5) in ihrem Grund (16) bezüglich Herstellung durch eine tangentiale Erzeugende (17) erreichbar sind.

9. Bandscheibenprothese nach einem der Ansprüche 1 bis 8, dadurch gekennzeichnet, dass die Aufnahmeflächen (2, 3) von ventral nach dorsal keilförmig aufeinander zulaufen.

10. Bandscheibenprothese nach einem der Ansprüche 1 bis 9, dadurch gekennzeichnet, dass die Aufnahmeflächen (2, 3) eine Strukturierung (19) für die Primärverankerung an den angrenzenden Wirbeln aufweisen.

11. Bandscheibenprothese nach einem der Ansprüche 1 bis 8, dadurch gekennzeichnet, dass mindestens Teile der Aufnahmeflächen (2, 3) konvex ausgeführt sind, um sich in intraoperativ erzeugten konkaven Gegenflächen der angrenzenden Wirbel zu zentrieren und zu verankern.

12. Bandscheibenprothese nach einem der Ansprüche 1 bis 9, dadurch gekennzeichnet, dass die Aufnahmeflächen (2, 3) Teile eines zum Nachbarwirbel vorstehenden Kragens (21) aufweisen, um ein Verrutschen der Bandscheibenprothese zu verhindern.

## Claims

1. An intervertebral disk prosthesis, which with respect to its external dimensions imitates a natural intervertebral disk, which in the implanted state in its axis (4) connects two adjacent vertebrae and which comprises an upper and a lower attachment surface (2, 3), whereby the intervertebral disk member (1) is in one piece and is made from a strong, elastically deformable material,
**characterised in that** the intervertebral disk member (1) comprises parallel, partially overlapping slits (5) at right angles to the axis (4), with adjacent slits (5) in an overlapping region (6) forming parts of leaf springs (7, 12, 13, 15) for the transmission of forces from the upper attachment surface (2) to the lower attachment surface (3).

2. An intervertebral disk prosthesis according to Claim 1,
**characterised in that** the slit width (10) and leaf spring thickness (11) are selected so that when determined pressure and/or bending loads are exceeded, the slit width (10) is reduced to zero at certain points and displacement restrictions are produced before the elastic limit is reached in the leaf springs.

3. An intervertebral disk prosthesis according to one of Claims 1 or 2,
**characterised in that** the strong, elastically deformable material is metal.

4. An intervertebral disk prosthesis according to one of Claims 1 to 3,
**characterised in that** the leaf spring (12) of one plane is clamped at three distinct locations.

5. An intervertebral disk prosthesis according to one of Claims 1 to 3,
**characterised in that** the leaf spring (13) of one plane is clamped at two distinct locations.

6. An intervertebral disk prosthesis according to one of Claims 1 to 3,
**characterised in that** the leaf spring (15) of one plane is clamped at one distinct location.

7. An intervertebral disk prosthesis according to one of Claims 1 to 6,
**characterised in that** the free ends (14) of leaf springs (7) act as spiral springs with the unilateral reduction of the slit width (10) to zero and act as displacement restrictions with the bilateral reduction of the slit width (10) to zero.

8. An intervertebral disk prosthesis according to one of Claims 1 to 6,
**characterised in that** with respect to manufacture the base (16) of slits (5) can be achieved by a tangential generatrix (17).

9. An intervertebral disk prosthesis according to one of Claims 1 to 8,
**characterised in that** the attachment surfaces (2, 3) converge in a wedge shape from the ventral to the dorsal end.

10. An intervertebral disk prosthesis according to one of Claims 1 to 9,
**characterised in that** the attachment surfaces (2, 3) have a structure (19) for the primary attachment to the adjacent vertebrae.

11. An intervertebral disk prosthesis according to one of Claims 1 to 8,
**characterised in that** at least parts of the attachment surfaces (2, 3) have a convex design in order to be centred and attached in intraoperatively produced concave counter-faces of the adjacent vertebrae.

12. An intervertebral disk prosthesis according to one of Claims 1 to 9,
**characterised in that** the attachment surfaces (2, 3) comprise parts of a collar (21) projecting to the adjacent vertebra to prevent slipping of the intervertebral disk prosthesis.

## Revendications

1. Prothèse de disque intervertébral, qui dans ses dimensions extérieures suit la forme d'un disque intervertébral naturel, qui à l'état implanté relie dans son axe (4) deux vertèbres voisines et qui présente une surface de logement supérieure (2) et une surface de logement inférieure (3), dans laquelle le corps (1) du disque intervertébral est en une partie et est réalisé dans un matériau solide, élastiquement déformable, caractérisée en ce que le corps (1) du disque intervertébral présente transversalement à l'axe (4) des fentes (5) parallèles et se recouvrant partiellement, des fentes (5) voisines formant dans une zone de recouvrement (6) des parties de ressort à lames (7, 12, 13, 15) pour la transmission de forces de la surface de logement supérieure (2) à la surface de logement inférieure (3).

2. Prothèse de disque intervertébral selon la revendication 1, caractérisée en ce que la largeur de fente (10) et l'épaisseur de ressort à lames (11) sont choisies telles qu'en cas de dépassement de sollicitations en compression et/ou flexion déterminées, la largeur de fente (10) en des points déterminés est réduite à zéro et des limitations de distance se produisent avant que la limite d'élasticité ne soit atteinte dans les ressorts à lames.

3. Prothèse de disque intervertébral selon l'une des revendications 1 et 2, caractérisée en ce que le matériau solide, élastiquement déformable est un métal.

4. Prothèse de disque intervertébral selon l'une des revendications 1 à 3, caractérisée en ce que le ressort à lames (12) d'un plan est serré en trois points marqués.

5. Prothèse de disque intervertébral selon l'une des revendications 1 à 3, caractérisée en ce que le ressort à lames (13) d'un plan est serré en deux points marqués.

6. Prothèse de disque intervertébral selon l'une des revendications 1 et 2, caractérisée en ce que le ressort à lames (15) d'un plan est serré en un point marqué.

7. Prothèse de disque intervertébral selon l'une des revendications 1 à 6, caractérisée en ce que les extrémités libres (14) de ressorts à lames (7) agissent, en cas de réduction unilatérale à zéro de la largeur de fente (10), en tant que ressorts de flexion et, en cas de réduction bilatérale à zéro de la largeur de fente (10), en tant que limitation de distance.

8. Prothèse de disque intervertébral selon l'une des revendications 1 à 6, caractérisée en ce que des fentes (5) peuvent être atteintes dans leur fond (16) en ce qui concerne la fabrication, par une génératrice tangentielle (17).

9. Prothèse de disque intervertébral selon l'une des revendications 1 à 8, caractérisée en ce que les surfaces de logement (2, 3) convergent l'une vers l'autre en forme de coin, du côté ventral au côté dorsal.

10. Prothèse de disque intervertébral selon l'une des revendications 1 à 9, caractérisée en ce que les surfaces de logement (2, 3) présentent une structuration (19) pour l'ancrage primaire sur les vertèbres adjacentes.

11. Prothèse de disque intervertébral selon l'une des revendications 1 à 8, caractérisée en ce qu'au moins des parties de surfaces de logement (2, 3) sont convexes, pour se centrer et s'ancrer dans des contre-surfaces concaves, produites au cours d'une opération, des vertèbres adjacentes.

12. Prothèse de disque intervertébral selon l'une des revendications 1 à 9, caractérisée en ce que les surfaces de logement (2, 3) présentent des parties d'un collet (21) faisant saillie vers la vertèbre voisine, pour empêcher un glissement de la prothèse de disque intervertébral.
